# EUROPEAN PATENT APPLICATION

(11) **EP 1 148 562 A1**
(43) Date of publication of application: **24.10.2001**
(21) Application number: 01303584.5
(22) Date of filing: 19.04.2001
(51) Int. Cl.: H01M 2/04

(54) **Unitary lid for an electrochemical cell**

(30) Priority: 19.04.2000 US 198175 P
(71) Applicant: Wilson Greatbatch Limited, Clarence New York 14031 (US)
(72) Inventor: Warchocki, David, North Tonawanda, NY 14120 (US); Carroll, James, Buffalo, NY 14220 (US); Quattrini, Paul J., Amherst, NY 14226 (US); McNamara, George, Amherst, NY 14226 (US)
(74) Representative: Bradley, Josephine Mary

(57) **Abstract**

A unitary lid for the casing of an electrochemical, is described. The lid has a terminal lead ferrule and a fillport formed from a single blank in a machining process. The lid does not require any welding except for securing it to the open end of a casing container. This helps the lid contribute to the cell's volumetric efficiency, which is especially important for cells powering implantable medical device.

## Description

### CROSS-REFERENCE TO RELATED APPLICATION

Applicant hereby claims priority based on U.S. Provisional Application No. 60/198,175 filed April 19, 2000.

### FIELD OF INVENTION

The present invention relates to electrochemical cells, and more particularly to covers of housings for electrochemical cells.

### BACKGROUND OF THE INVENTION

Electrochemical cells typically include a container with an opening which is closed by a lid or cover welded to the container to form a casing for the cell. Inside the container is an anode/cathode electrode assembly. The container and the cover are of electrically conductive material and serve as a contact for either the anode electrode or the cathode electrode. In a case positive cell, the cathode current collector is in contact with the casing while for a case negative design, the anode electrode is in contact with the cover and container. The other of the anode electrode and the cathode electrode not in contact with the casing is connected to a terminal lead electrically insulated from the casing by a glass-to-metal seal. Further, the casing contains an electrolyte for activating the cathode/anode electrode assembly. When a load is connected to the casing and the terminal pin, a chemical reaction in the cell results in a voltage differential which generates an electrical current to power the load, for example, a medical device.

The lid must provide access to the interior of the casing for at least two purposes. First, the terminal lead connected to the anode or the cathode current collector must pass through one of the lid openings to a position exterior of the casing. Second, the electrolyte must be filled into the housing through the other lid opening. Conventionally, two openings are defined in the lid for this purpose. The openings usually have structures connected to the lid to aid in sealing them. For example, a terminal lead ferrule is attached to the lid to accommodate the electrical lead and a fillport/closure assembly is used for sealing the fill opening.

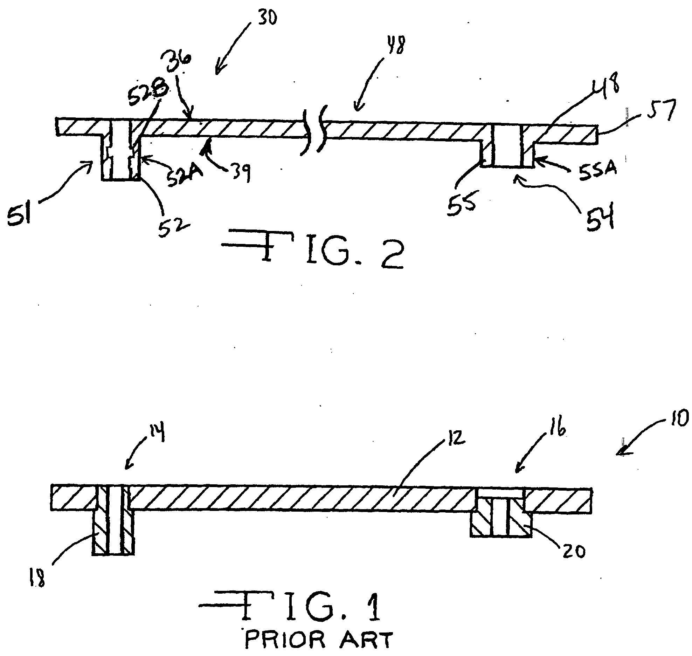

Fig. 1 shows an exemplary prior art construction where the lid 10 is formed of a generally rectangular blank 12 stamped from a sheet of electrically conductive material. During stamping, two openings 14, 16 are provided through the blank 12. A terminal lead ferrule 18 and a fillport 20 are sleeve-shaped members formed of discrete parts that are welded to the blank 12, each in registration with one of the openings 14, 16.

This prior art lid requires a number of manufacturing, inspection, and assembly steps due to the use of at least three discrete parts, i.e. the blank 12, the terminal lead ferrule 18 and the fillport 20. Specifically, the blank 12 is punched from a sheet of metal using a fine blanking or stamping operation. Simultaneously, the two openings 14, 16 are punched through the blank 12. The lid 10 goes through an annealing process, a passivation process (e.g. removal of free iron from the surface of the part) and a cleaning process before it is inspected. The discrete terminal lead ferrule 18 and the discrete fillport 20 go through the same process steps prior to attachment to the blank 12. The terminal lead ferrule 18 and the fillport 20 are then positioned in registration with the openings 14, 16 and welded thereto. These welds are vulnerable to variations in quality and each must be inspected. As those who are skilled in the art will readily recognize, these manufacturing, assembly, and inspection steps require time and labor, which add to the cost of an electrochemical cell. Also, inventory of the parts must be tracked and maintained, further adding to the cost of a cell.

Another problem with the prior art cover construction is that crevice corrosion can occur where the terminal lead ferrule 18 and the fillport 20 are secured to the openings 14, 16 in the lid 10. Typically, the terminal lead ferrule 18 and the fillport 20 are inserted from the bottom or interior surface of the blank 12 before being welded. This welding may leave cracks or crevices between the mating surfaces leading to entrapment of materials such as cleaning solutions. As such, corrosion can occur around these crevices.

Another prior art lid or cover for an electrochemical cell is described in U.S. Patent No. 6,010,803 to Heller, Jr. et al. This lid is formed by a metal injection molding process which requires that the intersections between the terminal lead ferrule and the main body of the lid and between the fillport structure and the lid be slightly curved or "radiused." Heller, Jr. et al. believe that radiused junctions facilitate the flow of material during the metal injection molding process. This eliminates areas of stress concentration which can cause the molded material to crack.

There are several problems with the Heller, Jr. et al. metal injection molded lid. First, the radiused areas detract from the internal volume available to active and other no-active cell components. It should be pointed out that electrochemical cells of the present invention are used to power implantable medical devices such as cochlear implants. These are extremely small devices which require extremely compact power sources where maximizing internal volume is important.

Secondly, a machined lid according to the present invention has a higher density and, consequently, less porosity than the metal injection molded lid. Metal injection molded materials require a binder, and even though technology advances have reduced the amount of binder required, metal density is still about 98% to about 98.5%, after curing. In contrast, a one piece lid according to the present invention machined from bar or rod stock has a density of about 99.99%, and maintains acceptable mechanical properties required for glassing the terminal lead in the glass-to-metal seal.

Lastly, design structures can be repositioned or changed to accommodate a particular cell. Typically, this requires a program change to offset features, change tolerances or add new features. In contrast, metal injection molded components require a whole new set of tooling which is capitol intensive.

Accordingly, what is needed is a unitary lid having a terminal ferrule and fillport structure that reduces the manufacturing, assembly, and inspection steps described above and is as compact in size as possible.

### SUMMARY OF THE INVENTION

The present invention meets the above-described needs by providing a unitary lid including a terminal ferrule and a fillport structure formed from a single blank of conductive material. A starting blank is provided with a thickness sufficient to meet the design features for a particular electrochemical cell. The terminal ferrule and fillport are then created in the blank via a machining process such that the junctions where both the terminal lead ferrule and the fillport structure meet with the under side of the lid are at right angles. The process of the present invention eliminates the need for welding and requires fewer handling operations while optimizing the cell's internal volume.

### BRIEF DESCRIPTION OF THE DRAWINGS

The invention is illustrated in the drawings in which like reference characters designate the same or similar parts throughout the figures of which:
Fig.1 is cross-sectional side elevation view of a conventional lid with a welded terminal lead ferrule and fillport structure;
Fig. 2 is a cross-sectional side elevation view of the unitary lid of the present invention; and,
Fig. 3 is a cross-sectional side elevation view of an electrochemical cell with the unitary lid of the present invention attached to a container to provide a casing for the electrode assembly.

### DETAILED DESCRIPTION OF THE EMBODIMENTS

Throughout this description the terms "lid" and "cover" are used interchangeably to refer to the member shown in Fig. 2 that is attached to the open end of a battery container or housing to form a casing for an electrochemical cell.

Electrochemical cells or batteries generate electrical current from chemical energy. Often, they are used as the power source for medical devices such as heart pacemakers and cochlear implants. In that light, the lid of the present invention is a compact unitary member with space saving right angle surfaces at the junction of the lid body and both the terminal ferrule and the fillport. As will be described hereinafter, this makes the present lid particularly applicable for cells intended to power implantable medical devices.

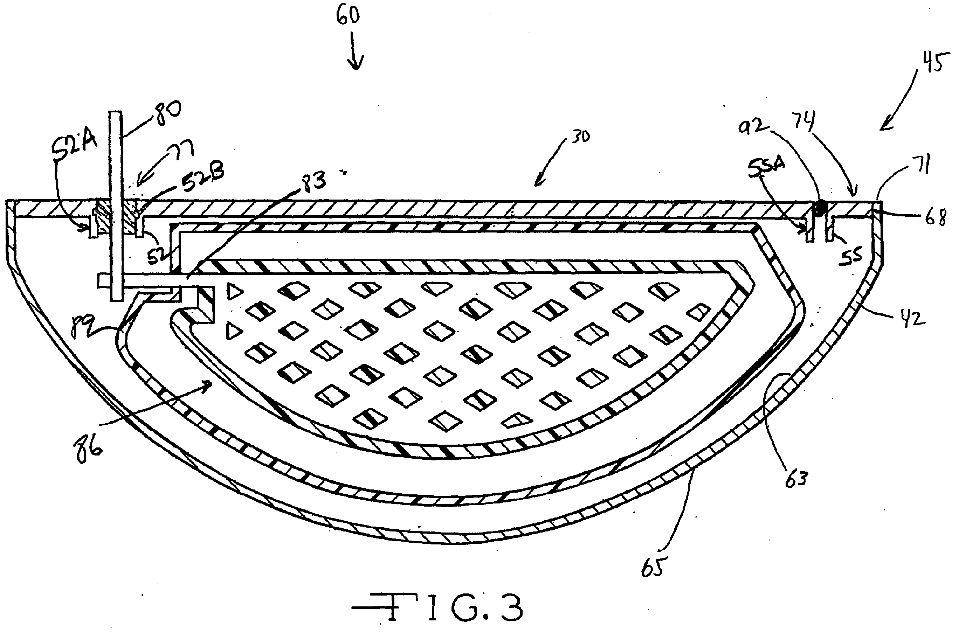

Turning now to the drawings, Fig. 2 shows a unitary lid or cover 30 according to the present invention formed by machining a rectangular blank (now shown) of an electrically conductive material such as stainless steel, titanium, nickel, aluminum and the like. Lid 30 has generally opposing major planar upper and lower surfaces 36 and 39. When in place closing the open end of the container 42 of a cell casing 45 (Fig. 3), upper surface 36 is an exterior surface and inner surface 39 is an interior surface. Lid 30 is formed of generally three portions or regions: a main body portion 48 having the opposed upper and lower surfaces 36, 39, a terminal ferrule portion 51, and a fillport portion 54. The terminal lead ferrule 51 and fillport portion 54 are completely integral or unitary with main body portion 48. Completely integral as used herein means being of a single continuous body of material. In other words, by machining the lid 30 from a suitable blank, the terminal ferrule 51 and the fillport portion 54 are not separate or discrete parts, but rather are completely unitary with the main body portion 48 forming a single part.

Lid 30 terminates along a peripheral edge 57 that is generally perpendicular to the planar upper and lower lid surfaces 36, 39. In the embodiment shown, main body portion 48 is generally rectangular in peripheral shape. The terminal ferrule 51 is a sleeve-shaped portion having a surrounding side wall 52 with a cylindrical outer surface 52A. An annular ring 52B is formed on the interior of the surrounding side wall. The annular ring 52B is formed from machine cutters and enhances the integrity of the glass-to-metal seal by providing stop-gaps or attachment structures for the glass of the glass-to-metal seal to fill and anchor into. With a prior art metal injection molded lid according to the previously discussed Heller, Jr. et al. patent, the provision of an inner annular ring provides structure that is horizontal to the longitudinal axis of the opening in the terminal ferrule. This creates opportunities for seal failure and weakening the seal structure. Also, the hoop strength of a lid produced by a metal injection molding process is reduced due to the extended curing time required.

The cylindrical outer surface 52A of the ferrule side wall 52 meets the lower surface 39 of the lid main body portion 48 at a right angle or a normal orientation. Similarly, the fillport 54 is a sleeve-shaped portion having a cylindrically-shaped opening provided by a surrounding side wall 55. The cylindrical outer surface 55A of the fillport side wall 55 meets the lower surface of the lid main body portion 48 at a right angle. If desired, the interior of the side wall 55 can be provided with an annular ring similar to ring 52B for the terminal ferrule.

While the upper end of the terminal ferrule 51 and the fillport 54 is shown co-planar with the upper surface 36 of the lid 30, the present invention should not be so limited. In that respect, these structures can extend above the upper lid surface 36. What is important is that they are unitary with the main body portion 48.

It will be understood by those of ordinary skill in the art that the main body portion 40 of the lid 30 according to this invention may be of any suitable shape to mate with and close an opening in a container 42 for a casing, which also may be of any suitable shape. Therefore, the present invention contemplates any configuration of two portions of a cell casing which when mated form a cavity therein. Either one of such portions of the casing, i.e., the lid or the container, may include the terminal ferrule portion 51 and the fillport portion 54 and be formed as a single part. What is important is that the respective outer surfaces 52A, 55A of the terminal ferrule 52 and the fillport 55 meet the lower or inner surface 39 of the lid main body portion 48 at a normal orientation. This means that as little internal casing volume as possible is occupied by the unitary lid 30. Such a construction benefits volumetric cell efficacy, which is especially important in cells intended to power implantable medical devices.

Fig. 3 shows an illustrative electrochemical cell 60 incorporating a lid or cover according to the present invention. The cell 60 is described in U.S. Patent No. 5,750,286 to Paulot et al., which is assigned to the assignee of the present invention and incorporated herein by reference. The cell 60 includes the casing 45 made of metal, such as stainless steel, titanium, nickel, aluminum, or other suitable electrically conductive material. Casing 45 is formed of two portions: the container shell 42 and the lid 30. Container 42 has an interior surface 63 and an opposite exterior surface 65. Further, container 45 terminates in a peripheral region 68 at a peripheral edge or rim 71. Peripheral region 68 defines an open side or opening 74 leading into the container 42. Accordingly, the container 42 forms generally all but one open side of casing 45. Lid 30 closes opening 74 and is attached to the peripheral region 68, such as by welding.

As shown in Fig. 3, the terminal ferrule 52 supports a glass-to-metal seal 77 for a terminal lead 80 connected to the current collector 83 of one of the electrode, for example the cathode electrode 86. The anode (not shown) is segregated from the cathode by a separator 89. The anode/cathode electrode assembly is then activated by an electrolyte (not shown) filled in the casing, and sealed therein by a closure means, such as ball 92 sealed in the fillport portion 54. Those skilled in the art will understand that the present invention is not limited to any particular closure structure.

In accordance with the previous description, it will be evident that the present invention is applicable to any electrochemical cell type in which a housing is used that has a container portion with an open side and a cover for closing the container, thereby forming a casing for the cell. The present invention is applicable to low rate, medium rate, high rate, case negative and case positive electrochemical cells of both primary and secondary chemistries. Examples of such cells include lithium iodine cells, lithium thionychloride cells, lithium silver vanadium oxide cells, lithium carbon monofluoride cells, lithium manganese dioxide cells, and secondary cells containing lithium cobalt oxide, and the like.

It will further be recognized that such cells may take one of various configurations. For example, depending on the type of cell, the configuration of the anodes, cathodes, terminal lead ferrule portions, fillports, etc. will vary. Also, for example, depending on the cell, the materials housed in the casing will vary. Such materials may take the form of a liquid or a solid depending on the type of cell. Therefore, it should be clear that the present invention is in no manner limited to the illustrative cell described herein and is applicable to all types of electrochemical cells.

While the invention has been described in connection with certain embodiments, it is not intended to limit the scope of the invention to the particular forms set forth, but, on the contrary, it is intended to cover such alternatives, modifications, and equivalents as may be included within the spirit and scope of the invention as defined by the appended claims.

## Claims

1. An electrochemical cell, which comprises:
a) a container having a surrounding side wall providing an opening leading into the container;
b) an electrode assembly comprising an anode and a cathode in an electrochemical association with each other and disposed inside the container;
c) a lid having space apart upper and lower surfaces joined by a pheripheral edge and secured to the open end of the container to provide a casing housing the electrode assembly, wherein the lid has at least a unitary terminal ferrule extending below the lid lower surface with a first outer surface of the terminal ferrule in a normal orientation therewith;
d) a terminal lead connected to one of the anode and cathode electrodes and extending through the terminal ferrule to a position spaced above the upper surface of the lid, wherein the terminal lead is sealed in the terminal ferrule in an insulated relationship with the housing; and
e) an electrolyte provided in the casing to activate the anode and cathode electrodes.

2. The electrochemical cell of claim 1 wherein the first outer surface of the terminal ferrule is cylindrical.

3. The electrochemical cell of claim 1 wherein the lid has a unitary fill port extending below the lid lower surface with a second outer surface of the fill port in a normal orientation therewith.

4. The electrochemical cell of claim 3 wherein the second outer surface of the fillport is cylindrical.

5. The electrochemical cell of claim 1 wherein the lid is of a conductive material.

6. The electrochemical cell of claim 1 wherein the conductive material is selected from the group consisting of stainless steel, titanium, nickel and aluminum.

7. The electrochemical cell of claim 1 wherein the lid is characterized as having been provided by a machinery process.

8. The electrochemical cell of claim 1 wherein the terminal ferrule includes a cylindrically shaped inner wall surrounding an opening in the terminal ferrule.

9. The electrochemical cell of claim 8 wherein the inner wall is provided with an annular recess.

10. A method for providing a lid for a casing of an electrochemical cell, comprising the steps of:
a) obtaining a blank; and
b) machining the blank to provide the lid having spaced apart upper and lower surfaces joined by a peripheral edge with at least a unitary terminal ferrule extending below the lid lower surface, wherein a first outer surface of the terminal ferrule is in a normal orientation with the lid lower surface.

11. The method of claim 10 wherein the first outer surface of the terminal ferrule is cylindrical.

12. The method of claim 10 including machining the blank having a unitary fillport extending below the lid lower surface with a second outer surface of the fillport in a normal orientation therewith.

13. The method of claim 10 including machining the terminal ferrule having a cylindrically shaped inner wall surrounding an opening in the terminal ferrule.

14. The method of claim 13 wherein the inner wall is provided with an annular recess.

15. The method of claim 10 wherein the second outer surface of the fillport is cylindrical.

16. The method of claim 10 including providing the lid of a conductive material.

17. A method for providing an electrochemical cell, comprising the step of:
a) providing a container having a surrounding side wall with an opening leading into the container;
b) disposing an electrode assembly comprising an anode and a cathode in an electrochemical association with each other inside the container;
c) machining a blank to provide a lid having spaced apart upper and lower surfaces joined by a peripheral edge and at least a unitary terminal ferrule extending below the lid lower surface, wherein a first outer surface of the terminal ferrule is in a normal orientation with the lid lower surface;
d) connecting a terminal lead to one of the anode and cathode electrodes;
e) securing the lid to the container to close the opening leading therein and thereby providing a casing for the cell with the terminal lead extending through the terminal ferrule to a position spaced above the upper surface of the lid and with the terminal lead insulated from the lid; and
f) activating the anode and cathode electrodes with an electrolyte provided in the casing.

18. The method of claim 17 wherein the first outer surface of the terminal ferrule is cylindrical.

19. The method of claim 17 including machining the blank having a unitary fillport extending below the lid lower surface with a second outer surface of the fillport in a normal orientation therewith.

20. The method of claim 17 wherein the second outer surface of the fillport is cylindrical.
